# EUROPÄISCHE PATENTANMELDUNG

(11) **EP 0 995 444 A2**
(43) Veröffentlichungstag der Anmeldung: **26.04.2000**
(21) Anmeldenummer: 99114435.3
(22) Anmeldetag: 22.07.1999
(51) Int. Cl.: A61K 38/11

(54) **Systematische Therapie von Schmerzen und depressiver Befindlichkeit**

(30) Priorität: 07.09.1998 DE 19840758
(71) Anmelder: pharmed Dr. Liedtke GmbH, 82027 Grünwald b. München (DE)
(72) Erfinder: Liedtke, Rainer K., Dr., 82027 Grünwald b. München (DE)
(74) Vertreter: Oser, Andreas

(57) **Zusammenfassung**

Zur systemischen Therapie von Schmerz und depressiver Befindlichkeit werden nichtinvasive Oxytocin Arzeiformen therapeutisch geeigneter Dosen eingesetzt. Die Therapie von Schmerz und depressiver Befindlichkeit ist für das Neuropeptid Oxytocin eine neue medizinische Indikation und eine neue pharmazeutisch-biologische Behandlungsform.

## Beschreibung

Die Erfindung betrifft eine systemische nichtinvasive Therapie von Schmerzen und depressiver Befindlichkeit mit Oxytocin Arzneiformen.

Es ist bekannt, daß Gewebsirritationen unterschiedlicher Ursache über sensorische Nervenstrukturen aufgenommen werden, am Entstehungsort neuronal als Schmerzsignale codiert und nach Fortleitung über nachfolgende neuronale Verschaltungen als biologische Information auf verschiedenen Ebenen des Zentralnervensystems (ZNS) aufgenommen und verarbeitet werden. Nach Interpretation der Signale im ZNS erfolgt dort Auslösung biologischer Reaktionen, die sowohl neuronal wie auch biochemisch hormonell sein können. Die regelungstechnischen Prinzipien entsprechen technisch einem interaktiven biologischen Netzwerk, einem biologischen Internet. Einfluss auf den Ausprägungsgrad von Schmerzen können sowohl materielle als auch primär ohne materiell erkennbares Substrat ablaufende Prozesse haben. Primär materielle Vorgänge sind z.B. Infektionen, Entzündungen, immunologische Vorgänge, physikalische Faktoren oder metabolische Störungen der Nervenbahnen selbst, primär immateriell sind emotionale Befindlichkeitsstörungen, die von verschiedenen Stressfaktoren abhängen und bei denen ein materielles Substrat nicht erkennbar ist.

Psychische Stimmungen und emotionale Befindlichkeitsstörungen wirken vorzugsweise auf der informatorisch komplexen Verarbeitungsebene der Interpretation perzeptierter Phänomene, worüber sich ein modulierender Einfluss auf die Schmerzwahrnehmung insgesamt ergibt.

Es ist bekannt, daß positiv gefärbte Stimmungslagen zu subjektiven Unterbewertungen negativer Ereignisse somit auch der von Schmerzsymptomatiken führen können obgleich die Ursachen objektiv schmerzhaft sind. Hierbei spielt vermutlich Ausschüttung körpereigener Schmerzstiller, Z.B. der Endorphine, eine Rolle. Bei negativ gedrückter Stimmungslage oder depressiver Befindlichkeit, deren Ursachen z.B. materiell hormonell oder psychosomatisch sein können, sind negativierte Interpretationen der perzeptierten Phänomene erkennbar. Bereits gerichtete Antizipationen gegenüber einem erwarteten Schmerzphänomen können zu reaktiven Über- oder Unterbewertungen führen (Pilkowsky, N.: in Wall P.D., Melzack, R., Textbook of Pain (1989); Churchill Livingstone; Edinburg, S. 980ff.). Umgekehrt sind depressive Befindlichkeiten auch Folge schmerzhafter Ereignisse, wobei sich beide Phänomene gegenseitig verstärken können und depressive Befindlichkeiten sogar physischen Schmerz bewirken können (Merskey, H., Boyd D., Pain: 5 (1978) 173).

Die Therapie von Schmerzsymptomatiken wird derzeit überwiegend gegen primär materielle Ereignisse gerichtet angegangen. Sie erfolgt pharmakologisch daher vorrangig mit nichtopioiden Analgetika, sogenannten nichsteroidalen Antiphlogistika (NSAIDs), Z.B. Diclofenac, Ibuprofen, mit Derivaten der Salicysäure, z.B. Acetylsaliclsäure, und mit Anilin-Derivaten, z.B. Paracetamol. In schweren Fällen erfolgt Einsatz von Opioiden, z.B. Pentazocin, Buprenorphin, Tramadal und in schwersten Fällen der von Opiaten (Brune K. Beck W. in: M. Zenz, I. Jura (Edits.) Lehrbuch der Schmerztherapie, WFG, Stuttgart 1993, S.121-135).
Weiterhin erfolgt verschiedentlich Einsatz sedierender und muskelrelaxierender Tranquillantien der Benzodiazepin-Gruppe sowie in einigen Fällen auch von Antidepressiva, z.B tricylischen Antidpressiva.

Da sich depressive Befindlichkeiten auf eine Schmerzperception negativ auswirken können macht es erklärlich, daß Verbesserungen negativer Stimmungslagen eine Reduktion der Schmerzperzeption bewirken können. So ist der bei Opiaten bekannte euphorisierende Effekt vermutlich eine essentielle Komponente in der Schmerztherapie aber leider auch der wesentliche Aspekt zur Sucht und Abhängigkeit bei Gesunden. Auch die bei Antidepressiva mit stimmungsaufhellender Komponente gesehenen Schmerzsenkungen dürften eine indirekte Effektkomponente sein (Egbunike. I.G. Chaffee, B.J.: Pharmacotherapy 199(10)267-70).
Demgegenüber besitzen aber die derzeit üblicherweise eingesetzten peripheren Analgetika/Antiphlogistika keine primären Wirkungen auf die Befindlichkeit.
Die Schmerztherapien mit diesen rein synthetisch-chemischen Stoffen zeigen nicht nur in viele Fällen unzureichende Wirkungen sondern auch eine erhebliche Rate unerwünschter Wirkungen. Bei NSAIDs finden sich als Folge ihres antiproliferativen Wirkungsmechanismus im Vordergrund Magenstörungen bis hin zu Erosionen der Magen- und Darmschleimhaut oder auch konsekutive Blutungen. Beim schwächer wirksamen Paracetamol, das keine antiphlogistische Wirkkomponente hat, findet sich eine metabolische Belastung der Leber- und Nieren-Funktionen, insbesondere bei höheren Dosierungen oder im Zusammenhang mit Alkohol. Benzodiazepine und Antidepressiva beinhalten neber starker Sedierung zahlreiche neurologische Nebeneffekte. Die derzeit eingesetzten Opioide und Opiate zeigen neben unerwünschten Effekten auf Atmung und Darm-Motilität insbesondere eine hohe Suchtgefahr.

Ein vom therapeutischen Ansatzpunkt vorteilhafteres Prinzip einer Schmerztherapie wären geeignete Anwendungsformen eines biologischen Stoffes der physiologisch indirekt in Schmerzprozesse involviert ist, steuerbar einsetzbar ist und als körpereigene Substanz über gute Gewebeverträglichkeit verfügt.

Daher wurde unter der Fragestellung nach potentiell wechselseitiger Beeinflussungen von Stimmungslage und Schmerz indirekten analgetischen Wirkungen verschiedener biologischer Substanzen nachgegangen.

Das körpereigene Nonapeptid Oxytocin hat bisher weder in der Schmerztherapie noch in der Therapie depressiver Befindlichkeiten eine therapeutische Beachtung oder Anwendung gefunden. Es ist ein sogenanntes Neuropeptid mit Hauptsyntheseort im Hypothalamus und spielt partiell die Rolle eines Neurotransmitters und partiell die Rolle eines Hormons. Traditionell pharmakologisch bekannt ist, daß Oxytocin an der glatten Muskulatur des Uterus und der Milchdrüsen muskelkontrahierend wirkt. Der Uteruseffekt ist insbesondere zum Ende einer Schwangerschaft ausgeprägt. Daher wird Oxytocin therapeutisch zur Wehenauslösung bei der Geburt eingesetzt, desweiteren zur Lakatationshilfe bei Stillstörungen. Da Oxytocin als Peptid chemisch Eiweissstruktur hat und Peptide bei oraler Gabe im Magen-Darmtrakt inaktiviert werden, wird es zur Wirkungserzielung vorzugsweise über die invasive Form der Injektion verabreicht.

Das biologische Effektspektrum von Oxytocin wurde erst in den letzten Jahren weiter detailliert untersucht und ist überaus komplex. Es weist basale biologische Effekte im Zentralnervensytem, im Endokrinium und inbesondere Einflüsse im sexuellen Verhaltens- und Stimmungsbereich auf (e.g. J.J. Evans: European Journal of Endocrinology;137 (1997) 559-571).

Die erhebliche Bandbreite lässt auf Anhieb kein pharmakologisch einheitliches Prinzip erkennen und beinhaltet sogar dynamische Effektwechsel. Da Oxytocin ein in Gesamtvorgänge eingebettetes physiologisches Prinzip ist, ist es zudem auch nicht isolierten pharmakologischen Wirkungsbetrachtungen zugänglich wie chemischsynthetische Pharmaka. Es ist somit nicht überraschend, daß sich daher bisher hier auch noch keine Formulierung für ein diesbezüglich praktisch anwendbares Therapiekonzept fand.

In weiterer Untersuchung der Frage gegenseitiger Beeinflussungen von Schmerz und Befindlichkeiten wurde der Frage potentieller analgetischer Wirkungen des Oxytocin nachgegangen. Tatsächlich zeigten verschiedene experimentelle Studien, daß bei invasiven Applikation über Injektion (intrathecal, intraperitoneal) auch Ocytocin-Effekte auf Nociception und am endogenen Opioid System (Arletti, R; Benelli, A; Bartolini, A.: Neuropeptides, 24(1993) 125-9), Lundeberg, T., Uvnaes-Moberg, K., Agren G., Bruzelius, G.: Neuro-science Letters 170 (1994) 153-7), Yang, J: Spine 19 (1994) 867-71) auftraten. Bei Kindern mit recurrent nichtorganischen Bauchschmerzen fanden sich erniedrigte Oxytocin Plasma Spiegel (Alfven, G., de la Torre, B., Uvnaes-Moberg, K.: Acta Paediatrica 83 (1994) 1075-80) und auch bei einer Gruppe depressiver Patienten fanden sich Hinweise auf erniedrigtes Plasma Oxytocin (Frasch, A., Zetscher, T., Steiger, A., Jirikowsksi, G.E.: Advances in Experimental Medicine and Biology 395 (1995) 257-258).

Auch aus diesen Einzelbefunden lässt sich noch nicht ein pharmakologisches Prinzip für eine diesbezügliche therapeutische Anwendung erkennen. Erst die Ermittlung und Erstellung einer biologischen Gesamtstrategie aus den unterschiedlichen experimentellen Einzeldaten ermöglichte es für Oxytocin ein diesbezüglich verwendbares Therapiekonzept abzuleiten:
Alle derzeit bekannten Oxytocin-Effekte zielen entweder auf Förderung oder Schutz reproduktionsphysiologischer Vorgänge. Operativ erfolgen die Oxytocin Effekte dabei dual, neuronal oder humoral und werden durch andere Hormone, insbesondere Steroidhormone kontrolliert, z.B. über Östrogene fördernd und über Gestagene hemmend. Diese hormonellen Kontrollen verlaufen aber ihrerseits phasenhaft in Zyklen, die den aktuellen Status des Reproduktionsgeschehens wiederspiegeln. Daher finden sich in direkter Abhängigkeit hierzu auch zu verschiedenen Zeiten unterschiedliche Ausprägungen von Oxytocin-Rezeptoren in den Zielorganen dieser Hormone.

Dies führt dann, sekundär und in direkter Beziehung zur kontrollierenden Hormonphase, zu unterschiedlichen Ausprägungen pharmakodynamischer Oxytocin-Effekte wie u.a. Muskelkontraktionen, Schmerzperception, Stimmungslage etc. und erklärt, zeitlich wechselnde Dosis-Wirkungs Beziehungen von Ocytocin an gleichen Zielorganen.
Da je nach Reproduktionsphase wechselnde Zielorgane hormonell für Oxytocin aktiviert sind, zeigt Oxytocin seine Effekte primär an den gleichen aktivierten Organen, u.a. z.B. neuronales ZNS Arousal zur Erreichung und Unterstützung der Konzeption, muskuläre Beeinflussung des Uterus zum Zeitpunkt der Geburt, humorale Hemmung der Stressachse zu Stillphasen und Beeinflussung der Milchdrüsenfunktion. Der in allen Phasen durch Oxytocin-Effekte dabei direkt bewirkte reziproke Ausgleich zwischen involvierten Schmerzphänomenen und positiver Stimmungslage spielt eine essentielle Rolle, da erst dieser biologische Basiseffekt das Reproduktionsziel dauerhaft fördert und unterstützt.

Der Erfindung liegt die Aufgabe zugrunde die nichtinvasive Therapie von Schmerzen und depressiver Befindlichkeit zu erweitern und verbessern.

Diese Aufgabe wird dadurch gelöst, daß nichtinvasive Arzneiformen mit Oxytocin eingesetzt werden, die mit therapeutisch geeigneten Dosen Oxytocin beladen sind mit denen Schmerzen und depressive Befindlichkeit verbessert werden, die technische Schutzmassnahmen gegen einen Abbau von Oxytocin besitzen, die systemisch wirksame Freisetzungen von Oxytocin ermöglichen und die Arzneiformen orale, rektale, vaginale, nasale, buccale, pulmonale, transdermale, oculare Arzneiformen sind.

Um die praktischen Therapiemöglichkeiten zu erweitern, umfasst, in einer weiteren Ausbildung der Erfindung, die neue medizinische Oxytocin Indikation Schmerzen und depressive Befindlichkeit alle Schmerzen oder depressiven Befindlichkeiten, die, durch sexuelle Dysfunktionen, menstruelle Störungen, Zyklusstörungen, postpartale Depressionen, weibliche Unterleibs- und Beckenbeschwerden, Störungen und Erkrankungen der weiblichen Brust, uterine Dysfunktionen oder Beschwerden, vaginale Dysfunktionen oder Beschwerden, Coitusbeschwerden, Libidostörungen, männliche erektile Dysfunktionen, Prostatabeschwerden bedingt sind.

Um die praktischen Therapiemöglichkeiten zu erweitern umfasst, in einer weiteren Ausbildung der Erfindung, die neue medizinische Oxytocin Indikation Schmerzen und depressive Befindlichkeit alle depressiven Befindlichkeiten, die durch Östrogene, Gestagene, Androgene, Gonadotropine, Prostaglandine oder Prolaktin bedingt sind.

Um die praktischen Therapiemöglichkeiten zu erweitern umfasst, in einer weiteren Ausbildung der Erfindung, die neue medizinische Oxytocin Indikation Schmerzen und depressive Befindlichkeiten alle depressiven Befindlichkeiten die durch Insulin und Glukagon bedingt sind und Gewichtstörungen beinhalten.

Um die praktischen Therapiemöglichkeit zu erweitern umfasst, in einer weiteren Ausbildung der Erfindung, die neue medizinische Oxytocin Indikation Schmerzen oder depressive Befindlichkeit alle depressiven Befindlichkeiten die über die Hypophysen-Nebennieren Stressachse durch Adrenalin, Noradrenalin, ACTH, CRH, Cortison bedingt sind und Angstgefühle und/oder Schlafstörungen beinhalten.

Um die praktischen Therapiemöglichkeiten zu erweitern sind die eingesetzten vaginalen und rektalen Arzneiformen Suppositorien, Globuli oder Klistiere.

Um die praktischen Therapiemöglichkeiten zu erweitern sind die eingesetzten transdermalen Systeme Matrixsysteme oder Laminat-Systeme.

Um die praktischen Therapiemöglichkeiten zu erweitern sind, in einer weiteren Ausbildung der Erfindung, die nasalen, pulmonalen oder buccalen Arzneiformen Micropartikel, Lösungen, Pulver, Spray oder Aerosole deren Anwendung als wiederholt verabreichbare Einzeldosen erfolgen kann.

Um die praktischen Therapiemöglichkeiten zu erweitern sind, in einer weiteren Ausbildung der Erfindung, die oralen Anwendungsformen Tabletten, Kapseln, Dragees, Granulate, Saft, Sirupe oder Lösungen.

Um die praktische Therapiemöglichkeiten zu erweitern, sind, in einer weiteren Ausbildung der Erfindung, chemisch modifizierte Analoge des natürlichen Oxytocins eingesetzt, wobei dies auch terminal COOH-extendierte Formen und N-a-Acetyl Oxytocin einschliesst.

Um die pharmazeutisch-technischen Anwendung zu erweitern, ist in einer weiteren Ausbildung der Erfindung, die eingesetzten Arzneiform aus biologisch verwertbaren oder biologisch abbaubaren Stoffen ausgebildet ist, wobei die biologischen Materialien Proteine oder Proteide, Lipide oder Lipoide, Kohlenhydrate oder Polysaccharide, oder Mischungen aus mehreren solcher Materialien sind.

Eine Therapie von Schmerz und depressiver Befindlichkeiten mittels der Anwendung nichtinvasiver Arzneiformen von Oxytocin wurde bisher nicht beschrieben und stellt ein neues biologisches Behandlungsprinzip dar.
Als Vorteil ergibt sich eine duale Therapie von Schmerzen und depressiver Befindlichkeit mit pharmazeutischer Verabreichung nur eines körpereigenen Stoffes. Dies wird möglich, da sich die Ausrichtung des primären Therapieeffektes durch die verabreichte Oxytocin Arzneiform aus der reziproken Wechselwirkung von Schmerzphänomen und Befindlichkeit als wechselseitig wirksamen Komponenten ergibt. Das hier pharmazeutisch genutzte biologische Prinzip eröffnet insbesondere im Bereich der Endokrinologie und Befindlichkeitsstörungen neue medizinische Anwendungsfelder, die je nach Ausgangslage, primär eine Therapiekomponente des Schmerz oder der depressiven Befindlichkeit darstellen.
Da es sich um einen körpereigenen Stoff handelt liegt auch eine höhere Sicherheit und Verträglichkeit vor als bei systemischen Therapien mit chemisch-synthetischen Analgetika.
Da für Applikationformen übliche pharmazeutische Techniken verwendet werden können, die dem Fachmann auf diesem Gebiet vertraut sind, ist als weiterer Vorteil eine kostengünstige Herstellung möglich. Da Oxytocin eine geringe biologische Halbwertszeit besitzt sind Effekte auch gut steuerbar.

Als ein Beispiel für eine technisch geeignet realisierbare vaginale oder rektale Arzeiform für die beschriebene Therapie mit Oxytocin, z.B. mit biologisch abbaubaren oder biologisch verwertbaren Materialien wird auf Lipid-Formulierungen verwiesen wie sie in US-Pat. 5120710, EP 0403748 oder JP 2-156611 beschrieben sind, ohne es auf diese beschriebene Technik zu beschränken. Diese Trägermaterialen sind auch für orale Anwendungen geeignet. Gundsätzlich können aber auch übliche Suppositorenmassen aus Mischungen mittelkettiger Triglyceride eingesetzt werden, z.B. Hartfettmischungen mit Witepsol H12, H 15, in die z.B. Dosierungen von 5 I.U. bis 200 I.U. Oxytocin eingebracht werden können.

Als Beispiel für eine technisch geeignet realisierbare Arzneiform für eine transdermale Anwendung im Rahmen der beschriebenen Therapie mit Oxytocin wird auf die Trägersysteme US-Pat. 4,765,986 und EP 0205974 verwiesen, ohne es auf diese Techniken beschränken zu wollen.
Als Beispiel für geeignet realisierbare Arzneiformen zur nasalen Anwendung für die beschriebene Therapie mit Oxytocin sind, ohne es auf diese beschriebene Technik zu beschränken, Nasensprays geeignet bei denen Oxytocin Z.B. in Konzentration von 40 I.U. pro ml vorliegt, bei denen als Vehikelbestandteile Chlorbutanol, Sorbitol, Parabene, Glycerol und Natriumhydrogenphosphat eingesetzt werden und mit denen über ca. 0,1 ml Einzeldosen ca. 4 I.U. in die Nasenhöhle eingebracht werden.

Zur systemischen Therapie von Schmerz und depressiver Befindlichkeit werden nichtinvasive Oxytocin Arzeiformen therapeutisch geeigneter Dosen eingesetzt. Die Therapie von Schmerz und depressiver Befindlichkeit ist für das Neuropeptid Oxytocin eine neue medizinische Indikation und eine neue pharmazeutisch-biologische Behandlungsform.

## Patentansprüche

1. Systemische Therapie von Schmerzen und depressiver Befindlichkeit, dadurch gekennzeichnet, daß nichtinvasive Arzneiformen mit Oxytocin eingesetzt werden, die mit den therapeutisch geeigneten Dosen Oxytocin beladen sind mit denen Schmerzen oder depressive Befindlichkeit verbessert werden, die technische Schutzmassnahmen gegen einen Abbau von Oxytocin besitzen, die systemisch wirksame Freisetzungen von Oxytocin ermöglichen, und die Arzneiformen orale, rektale, vaginale, nasale, buccale, pulmonale, transdermale, oculare Arzneiformen sind.

2. Systemische Therapie von Schmerzen und depressiver Befindlichkeit, nach Anspruch 1, dadurch gekennzeichnet, daß die neue medizinische Oxytocin Indikation Schmerzen und depressive Befindlichkeit alle Schmerzen oder depressiven Befindlichkeiten umfasst, die durch sexuelle Dysfunktionen, menstruelle Störungen, Zyklusstörungen, postpartale Depressionen, weibliche Unterleibs- und Beckenbeschwerden, Störungen und Erkrankungen der weiblichen Brust, uterine Dysfunktionen oder Beschwerden, vaginale Dysfunktionen oder Beschwerden, Coitusbeschwerden, Libidostörungen, männliche erektile Dysfunktionen, Prostatabeschwerden bedingt sind.

3. Systemische Therapie von Schmerzen und depressiver Befindlichkeit, nach vorhergehenden Ansprüchen, dadurch gekennzeichnet, daß die neue medizinische Oxytocin Indikation Schmerzen und depressive Befindlichkeit alle depressiven Befindlichkeiten umfasst, die durch Östrogene, Gestagene, Androgene, Gonadotropine, Prostaglandine oder Prolaktin bedingt sind.

4. Systemische Therapie von Schmerzen und depressiver Befindlichkeit, nach vorhergehenden Ansprüchen, dadurch gekennzeichnet, daß die neue medizinische Oxytocin Indikation Schmerzen und depressive Befindlichkeiten alle depressiven Befindlichkeiten die durch Insulin und Glukagon bedingt sind und Gewichtstörungen beinhalten.

5. Systemische Therapie von Schmerzen und depressiver Befindlichkeit, nach vorhergehenden Ansprüchen, dadurch gekennzeichnet, daß die neue medizinische Oxytocin Indikation Schmerzen oder depressive Befindlichkeit alle depressiven Befindlichkeiten die über die Hypophysen-Nebennieren Stressachse durch Adrenalin, Noradrenalin, ACTH, CRH, Cortison bedingt sind und Angstgefühle und/oder Schlafstörungen beinhalten.

6. Systemische Therapie von Schmerzen und depressiver Befindlichkeit, nach vorhergehenden Ansprüchen, dadurch gekennzeichnet, daß die eingesetzten vaginalen und rektalen Arzneiformen Suppositorien, Globuli oder Klistiere sind.

7. Systemische Therapie von Schmerzen und depressiver Befindlichkeit, nach vorhergehenden Ansprüchen, dadurch gekennzeichnet, daß die eingesetzten Transdermalsysteme Matrixsysteme oder Laminat-Systeme sind.

8. Systemische Therapie von Schmerzen und depressiver Befindlichkeit, nach vorhergehenden Ansprüchen, dadurch gekennzeichnet, daß die nasalen, pulmonalen oder buccalen Arzneiformen Micropartikel, Lösungen, Pulver, Sprays oder Aerosole sind mit denen Anwendung als wiederholt verabreichbare Einzeldosen erfolgen kann.

9. Systemische Therapie von Schmerzen und depressiver Befindlichkeit, nach vorhergehenden Ansprüchen, dadurch gekennzeichnet, daß die oralen Anwendungsformen Tabletten, Kapseln, Dragees, Granulate, Saft, Sirupe oder Lösungen sind.

10. Systemische Therapie von Schmerzen und depressiver Befindlichkeit, nach vorhergehenden Ansprüchen, dadurch gekennzeichnet, daß chemisch modifizierten Analoge des natürlichen Oxytocins eingesetzt sind, wobei dies auch terminal COOH-extendierte Formen und N-a-Acetyl Oxytocin einschliesst.

11. Systemische Therapie von Schmerzen und depressiver Befindlichkeit, nach vorhergehenden Ansprüchen, dadurch gekennzeichnet, daß die eingesetzte Arzneiform aus biologisch verwertbaren oder biologisch abbaubaren Stoffen ausgebildet ist, wobei die biologischen Materialien Proteine oder Proteide, Lipide oder Lipoide, Kohlenhydrate oder Polysaccharide, oder Mischungen aus mehreren solcher Materialien sind.
